# EUROPÄISCHE PATENTANMELDUNG

(11) **EP 2 025 352 A1**
(43) Veröffentlichungstag der Anmeldung: **18.02.2009**
(21) Anmeldenummer: 07114144.4
(22) Anmeldetag: 10.08.2007
(51) Int. Cl.: A61L 12/08, A61L 12/12

(54) **Verfahren zur Reinigung und Desinfektion einer Kontaktlinse mit oxidativen Reagenzien**

(71) Anmelder: Sturm, Albert, 50931 Köln (DE)
(72) Erfinder: Sturm, Albert, 50931 Köln (DE)
(74) Vertreter: von Kreisler Selting Werner

(57) **Zusammenfassung**

Verfahren zur Reinigung und Desinfektion einer Kontaktlinse mit oxidativen Reagenzien und Enzymen oder Enzymsystemen in wässriger Lösung, das folgende Schritte in der angegebenen Reihenfolge umfasst:
- Einbringen der Kontaktlinse in die wässrige Lösung,
- Einwirken lassen des Enzyms oder Enzymsystems auf die Kontaktlinse für eine Zeitdauer, die ausreicht, an der Kontaktlinse haftende Verunreinigungen proteinöser Herkunft mindestens teilweise zu spalten,
- nach dieser Zeitdauer die Enzym oder Enzymsysteme und Kontaktlinse enthaltende wässrige Lösung mit Ozon beaufschlagt wird und
- das System mit Ozon so lange behandelt wird wie zur Desinfektion der Kontaktlinse und der wässrigen Lösung notwendig.

## Beschreibung

Gegenstand der vorliegenden Erfindung ist ein Verfahren zur Reinigung und Desinfektion einer Kontaktlinse mit oxidativen Reagenzien sowie die Verwendung einer Vorrichtung zur Reinigung und Desinfektion einer Kontaktlinse.

Die Reinigung von insbesondere weichen Kontaktlinsen wird durch verschiedene Systeme gewährleistet. Weit verbreitet sind dabei wässrige Systeme, bei denen ein gelöstes oxidatives Reagenz auf die Kontaktlinse einwirkt und danach entfernt werden muss. Üblicherweise wird hierbei als oxidatives Reagenz aktiver Sauerstoff beispielsweise in Form von Wasserstoffperoxid oder Ozon eingesetzt. Im ersten Fall muss das überschüssige Wasserstoffperoxid durch die Zugabe anderer Reagenzien zerstört werden, um nicht versehentlich mit Wasserstoffperoxid kontaminierte Kontaktlinsen dem Anwender zuzuführen. Als Wasserstoffperoxid zerstörende Agentien werden Reduktionsmittel wie Natriumthiosulfat eingesetzt, die ihrerseits wiederum ausgespült werden müssen. Praktischer ist hier in der Anwendung der Einsatz von Ozon, da dann die umständliche Zerstörung des Wasserstoffperoxids unterbleiben kann.

Das europäische Patent EP 968 003 B1 offenbart eine effiziente Reinigung von Kontaktlinsen durch den simultanen Einsatz von Proteasen und Wasserstoffperoxid. Dabei zerstören Proteasen hartnäckige Proteinablagerungen in den Kontaktlinsen und das Wasserstoffperoxid desinfiziert die danach gereinigte Kontaktlinse. Nachteilig daran ist auch wiederum die Verwendung von Wasserstoffperoxid, das neutralisiert werden muss.

Weiterhin nachteilig an der Verwendung des Wasserstoffperoxids ist dessen Labilität, da es sich bei Lagerung langsam in Wasser und Sauerstoff zersetzt. Dies ist insbesondere in Ländern mit hohen Durchschnittstemperaturen zu beachten.

Wünschenswert ist mithin ein Verfahren zur Reinigung und Desinfektion einer Kontaktlinse, dass die oben geschilderten Nachteile vermeidet. Überraschenderweise wird diese Aufgabe erfindungsgemäß gelöst durch ein Verfahren zur Reinigung und Desinfektion einer Kontaktlinse mit oxidativen Reagenzien und Enzymen oder Enzymsystemen in wässriger Lösung, dass die folgenden Schritte in der Reihenfolge wie angegeben umfasst:
- Einbringen der Kontaktlinse in die wässrige Lösung,
- Einwirken lassen des Enzyms oder Enzymsystems auf die Kontaktlinse für eine Zeitdauer, die ausreicht an der Kontaktlinse haftende Verunreinigungen proteinöser Herkunft mindestens teilweise zu spalten, nach dieser Zeitdauer die Enzym- und Kontaktlinse enthaltende wässrige Lösung mit Ozon zu beaufschlagen und
- das System mit Ozon so lange zu behandeln, wie zur Desinfektion der Kontaktlinse und der wässrigen Lösung notwendig.

Das Ozon wird vorzugsweise mittels einer Vorrichtung, die von Yves Nold entwickelt worden ist und wie sie z.B. in US-A-5,051,137 beschrieben ist, erzeugt. Dadurch entfallen Lagerungen des oxidativen Reagenzes wie dies bei der Verwendung von Wasserstoffperoxid notwendig ist. Unzureichende Desinfektion durch überalterte Lösung des oxidativen Reagenzes werden somit vermieden. Als Enzym oder Enzymsystem kommen grundsätzlich alle Proteasen in Frage, die in der Lage sind, die typischerweise in Kontaktlinsen vorhandenen Proteinablagerungen zu hydrolysieren. Insbesondere geeignet sind hierbei Proteasen vom Typ des Subtilisins. Diese Proteasen sind im Stand der Technik umfangreich beschrieben, stellvertretend sei hier die EP-A-0 219 220 genannt. Die erfindungsgemäß verwendbaren Proteasen sind insbesondere Proteasen vom Serintyp und gehören zur Enzymklasse der alkalischen Proteasen.

Zur Einstellung eines im Wesentlichen augenphysiologischen Milieus kann die wässrige Lösung, in der die Kontaktlinse behandelt wird, Puffersubstanzen enthalten.

Erfindungsgemäß können gleichermaßen harte und weiche Kontaktlinsen behandelt werden. Typischerweise werden weiche Kontaktlinsen stärkerer Ablagerung in der Matrix ausgesetzt.

Es kann sich empfehlen, nach Herausnahme der Kontaktlinse aus der Reinigungslösung diese vor dem Einsetzen abzuspülen, um die Empfindlichkeit des jeweiligen Benutzers gegenüber Reizungen zu berücksichtigen.

Typischerweise beträgt die Zeitdauer des Einwirkenlassens des Enzyms oder Enzymsystems mindestens 30 min, insbesondere 60 min. Einwirkungszeiten von mehr als 3 h ändern regelmäßig nichts mehr am Ergebnis.

Je nach erzeugter Ozonmenge pro Zeiteinheit kann die Expositionsdauer variieren. Typischerweise beträgt die Expositionszeit der Kontaktlinse mit Ozon 30 min, insbesondere 60 min.

Das erfindungsgemäße Verfahren wird insbesondere in einer Vorrichtung ausgeführt, die dadurch gekennzeichnet ist, dass sie im Wesentlichen aus einem, eine Ionisationsröhre einschließenden Gehäuse aufweist, wobei die Ionisationsröhre mit einem Ende an eine Luftpumpe und mit dem anderen Ende an einer Ozonförderleitung angeschlossen ist. Die Ozonförderleitung ist mit einem Kapillarröhrchen versehen und weist ebenfalls eine elektrische Steuereinheit der Ionisationsröhre und der Luftpumpe auf. Des weiteren ist ein abnehmbarer Behälter vorgesehen, in den die Kontaktlinsenbehandlungslösung gegebenenfalls zusammen mit der Kontaktlinse angeordnet ist und in den das Kapillarröhrchen der Förderleitung hinein ragt. Es ist allerdings ebenfalls möglich, den Behälter in dem die Kontaktlinse behandelt wird auf den Ozongenerator zu stecken und durch eine geeignete Öffnung am Boden das Ozon hinzuleiten. Eine solche Vorrichtung nach Yves Nold ist z. B. in EP-B-0 346 553 beschrieben. Der Überdruck der durch die Freisetzung des aus dem Ozon gebildeten Sauerstoffs bewirkt wird, kann beispielsweise eine nicht ganz schließende Verschlusseinheit oder anderweitig gasdurchlässige Verschlusseinheit abgebaut werden, bzw. der Überdruck stellt sich nicht im nennenswerten Umfang ein.

## Patentansprüche

1. Verfahren zur Reinigung und Desinfektion einer Kontaktlinse mit oxidativen Reagenzien und Enzymen oder Enzymsystemen in wässriger Lösung, das folgende Schritte in der angegebenen Reihenfolge umfasst:
- Einbringen der Kontaktlinse in die wässrige Lösung,
- Einwirken lassen des Enzyms oder Enzymsystems auf die Kontaktlinse für eine Zeitdauer, die ausreicht, an der Kontaktlinse haftende Verunreinigungen proteinöser Herkunft mindestens teilweise zu spalten,
- nach dieser Zeitdauer die Enzym oder Enzymsysteme und Kontaktlinse enthaltende wässrige Lösung mit Ozon beaufschlagt wird und
- das System mit Ozon so lange behandelt wird wie zur Desinfektion der Kontaktlinse und der wässrigen Lösung notwendig.

2. Verfahren nach Anspruch 1, wobei das Enzym ausgewählt ist aus der Gruppe der Proteasen insbesondere Serinproteasen, alkalischen Proteasen.

3. Verfahren nach Anspruch 2, wobei das Enzym Subtilisin ist.

4. Verfahren nach einem der Ansprüche 1 bis 3, wobei die wässrige Lösung Puffersubstanzen enthält, zur Einstellung eines im Wesentlichen augenphysiologischen Milieus.

5. Verfahren nach einem der Ansprüche 1 bis 4, wobei als Kontaktlinsen harte und/oder weiche Kontaktlinsen gereinigt werden.

6. Verfahren nach einem der Ansprüche 1 bis 5, wobei nach Entnahme der Kontaktlinse nach Reinigung diese mit einer Lösung wie einer physiologischen Pufferlösung abgespült werden.

7. Verfahren nach einem der Ansprüche 1 bis 6, wobei die Zeitdauer des Einwirkenlassens des Enzyms oder Enzymsystems mindestens 30 min, insbesondere 60 min beträgt.

8. Verfahren nach einem der Ansprüche 1 bis 7, wobei die Zeit der Behandlung mit Ozon 30 min, insbesondere 60 min dauert.
